# EUROPEAN PATENT APPLICATION

(11) **EP 4 424 309 A1**
(43) Date of publication of application: **04.09.2024**
(21) Application number: 22887656.1
(22) Date of filing: 27.10.2022
(51) Int. Cl.: A61K 31/506, A61K 31/4184, A61K 31/7034, A61P 9/12

(54) **COMPOSITE COMPOSITION CONTAINING ANGIOTENSIN RECEPTOR BLOCKER AND SGLT2 INHIBITOR**

(30) Priority: 27.10.2021 KR 20210144625
(71) Applicant: Meditake Co., Ltd., Suwon-si, Gyeonggi-do 16710 (KR)
(72) Inventor: CHI, Yong Ha, Seogwipo-si, Jeju-do 63560 (KR)
(74) Representative: Gille Hrabal Partnerschaftsgesellschaft mbB Patentanwälte
(86) International application number: PCT/KR2022/016601
(87) International publication number: WO 2023/075461

(57) **Abstract**

The present invention relates to the combined administration of an angiotensin receptor blocker and an SGLT2 inhibitor for preventing or treating hypertension. The combined administration of the angiotensin receptor blocker and the SGLT2 inhibitor according to the present invention has a significantly excellent blood pressure-lowering effect compared to the administration of the angiotensin receptor blocker alone and can thus be effectively used as a treatment or combination for hypertension.

## Description

### [Technical Field]

The present invention relates to the combined administration of an angiotensin receptor blocker and a sodium-glucose co-transporter subtype 2 (SGLT2) inhibitor for preventing or treating hypertension.

### [Background Art]

Hypertension is a chronic degenerative disease of the circulatory system and is explained by the physiological mechanism of the renin-angiotensin system, which plays a vital role in the regulation of blood pressure and electrolyte balance. This mechanism involves the angiotensin-I converting enzyme (ACE) cleaving the dipeptide (His-Leu) from angiotensin-I, which is a decapeptide, to induce conversion into angiotensin-II, which has vasoconstrictive effects. The increase in angiotensin II produced by the angiotensin-converting enzyme has a strong blood pressure-raising effect and promotes the secretion of aldosterone, an antidiuretic hormone, and inhibits the excretion of water and sodium, which increases the volume of circulating blood, resulting in hypertension. Further, the angiotensin-converting enzyme is also responsible for breaking down and inactivating bradykinin, which has vasorelaxant properties, which in turn causes an increase in blood pressure.

Blood pressure-lowering drugs for the treatment of hypertension include diuretics, β-blockers, sympatholytics, vasodilators, Ca-antagonists, and angiotensin receptor blockers, of which the angiotensin receptor blockers (ARBs, angiotensin II antagonists) are known to lower blood pressure by preventing the binding of angiotensin II to its receptors on the blood vessel walls. However, many of these drugs may cause side effects such as increasing blood viscosity, causing electrolyte imbalances, triggering diabetes, worsening heart failure, decreasing energy, and impairing kidney function.

Hypertension is one of the most common cardiovascular diseases, and is typically diagnosed when the blood pressure is 140/90 mmHg. In recent years, the number of adult patients with hypertension has increased dramatically, and since hypertension may lead to acute heart disease or myocardial infarction, there is a continuous need to develop a more effective therapeutic agent for treating hypertension. Recently, combination drugs from different classes have been studied for the treatment of hypertension, but in some cases, these combination drugs may provoke interactions between the drugs, diminishing the desired effect or increasing the side effects associated with each medication. Accordingly, there is a need to develop combination drugs for the treatment of hypertension, including the choice of drug classes, dosage, combination formulation, dosage regimen, and the like.

Therefore, the present inventors aim to study a combination drug for the treatment of hypertension capable of enhancing the blood pressure-lowering effects of ARB class compounds while mitigating side effects thereof through dosage reduction.

### [Detailed Description of the Invention]

### [Technical Problem]

An object of the present invention is to provide a use for preventing or treating hypertension by the combined administration of an angiotensin receptor blocker and a sodium-glucose co-transporter subtype 2 (SGLT2) inhibitor.

Another object of the present invention is to provide a use for lowering blood pressure by the combined administration of an angiotensin receptor blocker and an SGLT2 inhibitor.

### [Technical Solution]

The present inventors has studied and endeavored to achieve the above objectives, and as a result, confirmed that the combined administration of an angiotensin receptor blocker and a sodium-glucose co-transporter subtype 2 (SGLT2) inhibitor significantly increases the blood pressure-lowering effect compared to the administration of the angiotensin receptor blocker alone, and completed the present invention.

In one aspect, the present invention provides a pharmaceutical composition for preventing or treating hypertension, comprising: (i) an angiotensin receptor blocker or pharmaceutically acceptable salts thereof; and (ii) an SGLT2 inhibitor or pharmaceutically acceptable salts thereof.

In another aspect, the present invention provides a composition for lowering blood pressure, comprising: (i) an angiotensin receptor blocker or pharmaceutically acceptable salts thereof; and (ii) an SGLT2 inhibitor or pharmaceutically acceptable salts thereof.

In the composition of the present invention, the angiotensin receptor blocker may be one or more selected from the group consisting of fimasartan, telmisartan, candesartan, azilsartan, olmesartan, and pharmaceutically acceptable salts thereof, and the SGLT2 inhibitor may be one or more selected from the group consisting of dapagliflozin, empagliflozin, and pharmaceutically acceptable salts thereof.

In still another aspect, the present invention provides a combination for preventing or treating hypertension comprising: (i) a first agent comprising an angiotensin receptor blocker or pharmaceutically acceptable salts thereof; and (ii) a second agent comprising an SGLT2 inhibitor or pharmaceutically acceptable salts thereof.

In the combination of the present invention, the first agent and the second agent may be administered either simultaneously or at different times. The combination of the present invention may be a composite preparation comprising the first agent and the second agent, specifically a composite preparation for oral administration.

In still another aspect, the present invention provides a pharmaceutical adjuvant composition for improving hypertension or lowering blood pressure of an angiotensin receptor blocker, comprising: an SGLT2 inhibitor or pharmaceutically acceptable salts thereof.

In still another aspect, the present invention provides a food composition for improving hypertension or lowering blood pressure, comprising: (i) an angiotensin receptor blocker or pharmaceutically acceptable salts thereof; and (ii) an SGLT2 inhibitor or pharmaceutically acceptable salts thereof.

In still another aspect, the present invention provides a method for preventing, treating, or improving hypertension, or lowering blood pressure, comprising: administering a therapeutically effective amount of (i) an angiotensin receptor blocker, or pharmaceutically acceptable salts thereof; and (ii) an SGLT2 inhibitor, or pharmaceutically acceptable salts thereof.

### [Advantageous Effects]

The combined administration of an angiotensin receptor blocker and a sodium-glucose co-transporter subtype 2 (SGLT2) inhibitor according to the present invention has a significantly excellent blood pressure-lowering effect compared to the administration of the angiotensin receptor blocker alone and can thus be effectively used as a treatment or combination drug for hypertension.

### [Description of Drawings]

FIG. 1 shows the dosage and schedule of administration of an angiotensin receptor blocker and a sodium-glucose co-transporter subtype 2 (SGLT2) inhibitor in a rat model of hypertension.
FIG. 2 is a graph showing the results of blood pressure measurements in the rat model of hypertension following administration of the angiotensin receptor blocker and the SGLT2 inhibitor.
FIG. 3 shows dosing schedule of the angiotensin receptor blocker and the SGLT2 inhibitor in the rat model of hypertension.
FIG. 4 is a graph showing the results of blood pressure measurements in the rat model of hypertension following administration of fimasartan and dapagliflozin.
FIG. 5 is a graph showing the results of blood pressure measurements in the rat model of hypertension following administration of telmisartan and dapagliflozin.
FIG. 6 is a graph showing the results of blood pressure measurements in the rat model of hypertension following administration of candesartan and dapagliflozin.
FIG. 7 is a graph showing the results of blood pressure measurements in the rat model of hypertension following administration of azilsartan and empagliflozin.
FIG. 8 is a graph showing the results of blood pressure measurements in the rat model of hypertension following administration of olmesartan and empagliflozin.
FIG. 9 is a graph showing the results of blood pressure measurements in the rat model of hypertension following administration of telmisartan and empagliflozin.

### [Best Mode]

Hereinafter, the present invention will be described in detail with reference to the accompanying drawings, which illustrate embodiments of the present invention. However, the following embodiments are presented as examples of the present invention, and detailed descriptions of techniques or configurations known to those skilled in the art may be omitted if it is determined that such detailed descriptions would unnecessarily obscure the gist of the present invention. The scope of the invention is not limited thereby. The present invention may be practiced in various modifications and applications within the equivalents described in the appended claims in below and construed therefrom.

In addition, the terminology used herein is intended to appropriately describe preferred embodiments of the present invention, which may vary depending on the user, the intention of the operator, or customary practice in the field to which the present invention belongs. Accordingly, definitions of these terms should be interpreted based on the context of this specification as a whole. Throughout the specification, when a portion is said to "include" a component, it means that it may further include other components, not exclude other components, unless specifically noted to the contrary.

All technical terms used in the present invention, unless otherwise defined, are used as commonly understood by one of ordinary skill in the relevant field of the present invention. In addition, even though preferred methods or samples are described herein, similar or equivalent ones are also encompassed within the scope of the present invention. The contents of all referenced publications herein are incorporated by reference into the present invention.

The present inventors have confirmed that the combined administration of an angiotensin receptor blocker and a sodium-glucose co-transporter subtype 2 (SGLT2) inhibitor can significantly improve the treatment of hypertension compared to the administration of angiotensin receptor blocker alone and exhibit similar blood pressure lowering effects at a smaller dose, and completed the present invention.

Thus, the present invention provides a pharmaceutical composition for preventing or treating hypertension, comprising: (i) an angiotensin receptor blocker or pharmaceutically acceptable salts thereof; and (ii) an SGLT2 inhibitor or pharmaceutically acceptable salts thereof.

As used herein, the term "angiotensin receptor blocker (ARB)" refers to an angiotensin II receptor blocker, and more specifically to an agent that inhibits the binding of angiotensin II to its receptor. The above angiotensin II receptor blocker is known to inhibit the binding of angiotensin II to an angiotensin II receptor on a cell membrane, the angiotensin II being a factor that causes elevated blood pressure, left ventricular hypertrophy, vascular hypertrophy, atherosclerosis, renal failure, or stroke.

The angiotensin receptor blocker may be selected from the group consisting of valsartan, telmisartan, losartan, candesartan, eprosartan, olmesartan, irbesartan, azilsartan, or fimasartan, and more particularly one or more selected from the group consisting of fimasartan, telmisartan, candesartan, azilsartan, olmesartan, and pharmaceutically acceptable salts thereof.

As used in the present invention, the term "sodium-glucose co-transporter subtype 2 (SGLT2) inhibitor" is known to be used in the treatment of diabetes, such as by inhibiting SGLT2 in renal tubules to excrete glucose from the blood into the urine, thereby exhibiting a hypoglycemic effect. The SGLT2 inhibitor may be canagliflozin, dapagliflozin, empagliflozin, ertugliflozin, ipragliflozin, luseogliflozin, or tofogliflozin.

According to an embodiment of the present invention, the angiotensin receptor blocker may be one or more selected from the group consisting of fimasartan, telmisartan, candesartan, azilsartan, olmesartan, and pharmaceutically acceptable salts thereof, and the SGLT2 inhibitor may be one or more selected from the group consisting of dapagliflozin, empagliflozin, and pharmaceutically acceptable salts thereof.

In other words, the present invention provides a pharmaceutical composition for preventing or treating hypertension, comprising: (i) at least one angiotensin receptor blocker selected from the group consisting of fimasartan, telmisartan, candesartan, azilsartan, olmesartan, and pharmaceutically acceptable salts thereof; and (ii) at least one SGLT2 inhibitor selected from the group consisting of dapagliflozin, empagliflozin, and pharmaceutically acceptable salts thereof.

As used herein, the term "pharmaceutically acceptable salt" refers to any organic or inorganic addition salt of which concentration has effective action that is relatively non-toxic and harmless to patients in which side effects caused by these salts do not degrade the beneficial efficacy of pharmacologically active ingredients. The pharmaceutically acceptable salt includes salts derived from pharmaceutically acceptable acids or bases. The acid usable for the preparation of the pharmaceutically acceptable salt may be an inorganic acid or an organic acid. Examples of inorganic acids may include, but not limited to, hydrochloric acid, sulfuric acid, nitric acid, phosphoric acid, perchloric acid, bromic acid, and the like, and examples of organic acids may include, but not limited to, acetic acid, methanesulfonic acid, ethanesulfonic acid, p-toluenesulfonic acid, fumaric acid, maleic acid, malonic acid, phthalic acid, succinic acid, lactic acid, citric acid, citrate, gluconic acid, tartaric acid, salicylic acid, malic acid, oxalic acid, benzoic acid, embonic acid, aspartic acid, glutamic acid, and the like. In addition, amino acid addition bases prepared using natural amino acids such as alanine, glycine, and the like, may also be included in the pharmaceutically acceptable salts of the present invention. Further, the base capable of being used in the preparation of the pharmaceutically acceptable salt may be, for example, tris(hydroxymethyl)methylamine, dicyclohexylamine, and the like, but is not limited thereto.

In the pharmaceutical composition of the present invention, pharmaceutically acceptable salts of an active ingredient may also comprise hydrates or solvates of the active ingredient. The hydrate or solvate may be formed by dissolving the active ingredient in a water-miscible solvent such as methanol, ethanol, acetone, or 1,4-dioxane and then adding a free acid or free base, followed by crystallization or recrystallization, but the method of forming the hydrate or solvate is not limited thereto. For example, a pharmaceutically acceptable salt of fimasartan may be fimasartan potassium trihydrate (fimasartan·K·3H₂O), and a pharmaceutically acceptable salt of dapagliflozin may be dapagliflozin propanediol hydrate, but examples thereof are not limited thereto.

As used herein, hypertension refers to the condition of abnormally elevated blood pressure as a component of the metabolic syndrome and has the same meaning as that commonly used in the art.

In an embodiment of the present invention, it was found that combined administration (co-administration) of an SGLT2 inhibitor with an angiotensin receptor blocker in a rat model of hypertension is effective in lowering blood pressure, with a remarkably enhanced effect compared to therapeutic effects of angiotensin receptor blockers or SGLT2 inhibitors alone (Examples 2 to 4).

In an embodiment of the present invention, the pharmaceutical composition may be usefully utilized as a combination drug for preventing or treating hypertension by optimizing a weight ratio of (i) an angiotensin receptor blocker or pharmaceutically acceptable salts thereof; and (ii) an SGLT2 inhibitor or pharmaceutically acceptable salts thereof for the therapeutic purpose. With respect to a preferred weight ratio, the angiotensin receptor blocker may be, relative to 1 part by weight of the SGLT2 inhibitor or pharmaceutically acceptable salts thereof, 0.05 to 20 parts by weight, and specifically 0.05 parts by weight, 0.1 parts by weight, 0.2 parts by weight, 0.3 parts by weight, 0.4 parts by weight, 0.5 parts by weight, 0.6 parts by weight, 0.7 parts by weight, 0.8 parts by weight, 0.9 parts by weight, 1 part by weight, 1.2 parts by weight, 1.5 parts by weight, 2 parts by weight, 2.2 parts by weight, 2.5 parts by weight, 3 parts by weight, 3.2 parts by weight, 3.5 parts by weight, 4 parts by weight, 4.2 parts by weight, 4.5 parts by weight, 5 parts by weight, 5.5 parts by weight, 6 parts by weight, 6.5 parts by weight, 7 parts by weight, 7.5 parts by weight, 8 parts by weight, 8.5 parts by weight, 9 parts by weight, 9.5 parts by weight, 10 parts by weight, 11 parts by weight, 12 parts by weight, 13 parts by weight, 14 parts by weight, 15 parts by weight or 20 parts by weight. Depending on the specific treatment mode of hypertension, the present invention provides a pharmaceutical composition comprising (i) an angiotensin receptor blocker or pharmaceutically acceptable salts thereof; and (ii) an SGLT2 inhibitor or pharmaceutically acceptable salts thereof, having a weight ratio within the range of two preferred weight ratios, each defined by lower and upper limits, of the above listed (i) angiotensin receptor blocker or pharmaceutically acceptable salts thereof and (ii) SGLT2 inhibitor or pharmaceutically acceptable salts thereof.

The weight ratio of (i) the angiotensin receptor blocker (specifically, fimasartan, telmisartan, candesartan, azilsartan, olmesartan), or pharmaceutically acceptable salts thereof; and (ii) the SGLT2 inhibitor (specifically, dapagliflozin or empagliflozin) or pharmaceutically acceptable salts thereof contained in the pharmaceutical composition of the present invention may be 0. 5 : 1 to 15 : 1, more particularly 0.8 : 1 to 12 : 1 or 1 : 1 to 8 : 1.

According to an embodiment of the present invention, the weight ratio of (i) the angiotensin receptor blocker or pharmaceutically acceptable salts thereof; and (ii) dapagliflozin or pharmaceutically acceptable salts thereof may be 0.5 : 1 to 15 : 1, and specifically 0.8 : 1 to 12 : 1. For example, the weight ratio of (i) the angiotensin receptor blocker or pharmaceutically acceptable salts thereof; and (ii) dapagliflozin or pharmaceutically acceptable salts thereof may be 3 : 1 to 12 : 1 as a weight ratio of fimasartan and dapagliflozin, may be 2 : 1 to 8 : 1 as a weight ratio of telmisartan and dapagliflozin, or may be 0.8 : 1 to 3.2 : 1 as a weight ratio of candesartan and dapagliflozin.

According to another embodiment of the present invention, the weight ratio of (i) the angiotensin receptor blocker or pharmaceutically acceptable salts thereof; and (ii) empagliflozin or pharmaceutically acceptable salts thereof may be 0.5 : 1 to 10 : 1, and specifically 1 : 1 to 8 : 1. For example, the weight ratio of (i) the angiotensin receptor blocker or pharmaceutically acceptable salts thereof; and (ii) empagliflozin or pharmaceutically acceptable salts thereof may be 2 : 1 to 8 : 1 as a weight ratio of azilsartan and empagliflozin, may be 1 : 1 to 4 : 1 as a weight ratio of olmesartan and empagliflozin, or may be 1 : 1 to 8 : 1 as a weight ratio of telmisartan and empagliflozin.

In addition, the pharmaceutical composition of the present invention may comprise 0.05 mol to 20 mol of angiotensin receptor blocker per 1 mol of SGLT2 inhibitor contained therein, and specifically 0.05 mol, 0.1 mol, 0.15 mol, 0.2 mol, 0.25 mol, 0.3 mol, 0.35 mol, 0.4 mol, 0.45 mol, 0.5 mol, 0.55 mol, 0.6 mol, 0.65 mol, 0.7 mol, 0.75 mol, 0.8 mol, 0.85 mol, 0.9 mol, 0.95 mol, 1 mol, 1.2 mol, 1.5 mol, 2 mol, 2.2 mol, 2.5 mol, 3 mol, 3.2 mol, 3.5 mol, 4 mol, 4.5 mol, 5 mol, 5.5 mol, 6 mol, 6.5 mol, 7 mol, 8 mol, 9 mol, 10 mol, 12 mol, 15 mol, or 20 mol per 1 mol of SGLT2 inhibitor contained therein. Depending on the specific treatment mode of hypertension, the pharmaceutical composition of the present invention comprises (i) the angiotensin receptor blocker or pharmaceutically acceptable salts thereof; and (ii) the SGLT2 inhibitor or pharmaceutically acceptable salts thereof, having a molar ratio within the range of two preferred molar ratios listed above, each defined by lower and upper limits.

According to an embodiment of the present invention, a molar ratio of (i) the angiotensin receptor blocker or pharmaceutically acceptable salts thereof; and (ii) dapagliflozin or pharmaceutically acceptable salts thereof may be 0.5 : 1 to 12 : 1, and specifically 0.7 : 1 to 10 : 1. For example, the molar ratio of (i) the angiotensin receptor blocker or pharmaceutically acceptable salts thereof; and (ii) dapagliflozin or pharmaceutically acceptable salts thereof may be 2.4 : 1 to 9.8 : 1 as a molar ratio of fimasartan and dapagliflozin, may be 1.6 : 1 to 6.4 : 1 as a molar ratio of telmisartan and dapagliflozin, or may be 0.74 : 1 to 2.97 : 1 as a molar ratio of candesartan and dapagliflozin.

According to another embodiment of the present invention, the molar ratio of (i) the angiotensin receptor blocker or pharmaceutically acceptable salts thereof; and (ii) empagliflozin or pharmaceutically acceptable salts thereof may be 0.5 : 1 to 10 : 1, and specifically 0.8 : 1 to 8 : 1. For example, the molar ratio of (i) the angiotensin receptor blocker or pharmaceutically acceptable salts thereof; and (ii) empagliflozin or pharmaceutically acceptable salts thereof may be 2 : 1 to 7.9 : 1 as a molar ratio of azilsartan and empagliflozin, may be 0.8 : 1 to 3.2 : 1 as a molar ratio of olmesartan and empagliflozin, or may be 0.87 : 1 to 7 : 1 as a molar ratio of telmisartan and empagliflozin.

The pharmaceutical composition of the present invention may be administered in a therapeutically effective amount. The therapeutically effective amount refers to a drug dosage that effectively prevents or treats hypertension. The appropriate total daily dose may be determined by the physician within the scope of sound medical judgment. The specific therapeutically effective amount for a particular patient will preferably vary depending on a variety of factors, including the type and degree of response to be achieved, the type and amount of drugs to be combined administered, the specific composition including whether other formulations are used in some cases, the patient's age, weight, general health, sex and diet, time of administration, route of administration, and duration of treatment, and similar factors well known in the field of medicine.

The pharmaceutical composition of the present invention may be administered orally or parenterally, preferably orally. Furthermore, the pharmaceutical composition of the present invention may be used in combination with one or more therapeutic agents for treating hypertension.

For administration, the pharmaceutical composition of the present invention may comprise a pharmaceutically acceptable carrier, excipient, and/or diluent, and the like. Examples of the carrier, excipient and/or diluent may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil, but the carrier, excipient and/or diluent are not limited thereto.

The pharmaceutical composition of the present invention may be prepared into pharmaceutical dosage forms using methods well known in the art. In the preparation of a dosage form, the active ingredient may be mixed or diluted with a carrier, or enclosed within a containerized carrier. The pharmaceutical composition of the present invention may be prepared into dosage forms for oral administration, for example, as tablets, troches, lozenges, aqueous or oily suspensions, prepared powders or granules, emulsions, hard or soft capsules, syrups or elixirs.

In one aspect, the present invention provides a combination for preventing or treating hypertension comprising: (i) a first agent comprising an angiotensin receptor blocker or pharmaceutically acceptable salts thereof; and (ii) a second agent comprising an SGLT2 inhibitor or pharmaceutically acceptable salts thereof.

In the present invention, the term "combination" means a combination of two or more active substances in a formulation and a combination in the sense of individual formulations of active substances administered at stated intervals from each other in therapy. Thus, the term "combination", when described in relation to the present invention, includes the clinical realization of simultaneous administration of two or more therapeutically effective compounds.

In the combination of the present invention, the angiotensin receptor blocker or pharmaceutically acceptable salts thereof may be fimasartan, telmisartan, candesartan, azilsartan, or olmesartan, and the SGLT2 inhibitor may be dapagliflozin or empagliflozin. For example, the combination may comprise: (i) a first agent comprising an angiotensin receptor blocker or pharmaceutically acceptable salts thereof; and (ii) a second agent comprising dapagliflozin, empagliflozin, or pharmaceutically acceptable salts thereof.

In the combination of the present invention, the first agent and/or second agent may each be administered parenterally or orally, preferably orally.

In the combination of the present invention, the first agent and the second agent may be administered either simultaneously or at different times.

The combination of the present invention may be a composite preparation comprising the first agent and the second agent, specifically a composite preparation for oral administration.

In one aspect, the present invention provides a pharmaceutical adjuvant composition for preventing, treating, or improving hypertension, or lowering blood pressure of an angiotensin receptor blocker, comprising: an SGLT2 inhibitor or pharmaceutically acceptable salts thereof.

As used in the present invention, the term "adjuvant" refers to a use in which the drug administered as an adjuvant has a relatively low preventive or therapeutic effect when administered alone, but has a significantly improved preventive or therapeutic effect on hypertension when administered in combination with other antihypertensive drugs.

In the composition, the angiotensin receptor blocker may be fimasartan, telmisartan, candesartan, azilsartan, or olmesartan, and the SGLT2 inhibitor may be dapagliflozin or empagliflozin.

The composition of the present invention may be a pharmaceutical composition or a food composition.

In other words, in one aspect, the present invention provides a food composition for improving hypertension or lowering blood pressure, comprising: (i) an angiotensin receptor blocker or pharmaceutically acceptable salts thereof; and (ii) an SGLT2 inhibitor or pharmaceutically acceptable salts thereof.

When the composition of the present invention is used as a food composition, the angiotensin receptor blocker and SGLT2 inhibitor or pharmaceutically acceptable salts thereof may be added as is or may be used in combination with other foods or food ingredients, and may be utilized as appropriate in accordance with general methods of the art. The composition may comprise, in addition to the active ingredient, a food-acceptable dietary supplement, and the amount of the active ingredient mixed in the composition may be suitably determined according to the purpose of use (preventive, health or therapeutic treatment).

The food composition of the present invention may include health functional food. The term "health functional food" used herein refers to food prepared and processed in the form of tablets, capsules, powders, granules, liquids and pills using raw materials or ingredients having useful functionalities for the human body. Here, 'functionality' means to obtain useful effects for health purposes, such as nutrient control, physiological action, and the like, on the structure and function of the human body.

Furthermore, there is no limitation on the types of health food products in which the composition of the present invention is usable. Moreover, the composition comprising the angiotensin receptor blocker and/or SGLT2 inhibitor of the present invention as the active ingredient may be prepared by mixing known additives with other suitable auxiliary ingredients that may be contained in a health functional food depending on the choice of a person skilled in the art. Examples of foods capable of being added include meat, sausages, bread, chocolate, candy, snacks, confectionery, pizza, ramen, other noodles, chewing gum, dairy products including ice cream, various soups, beverages, teas, drinks, alcoholic beverages, and vitamin complexes, and may be prepared by adding the composition according to the present invention as a main ingredient to nectars, teas, jellies and juices, etc.

In one aspect, the present invention provides a method for preventing or treating hypertension, or lowering blood pressure, comprising: administering a therapeutically effective amount of (i) an angiotensin receptor blocker, or pharmaceutically acceptable salts thereof; and (ii) an SGLT2 inhibitor, or pharmaceutically acceptable salts thereof.

In another aspect, the present invention provides a use for preventing or treating hypertension, or lowering blood pressure, of (i) an angiotensin receptor blocker, or pharmaceutically acceptable salts thereof; and (ii) an SGLT2 inhibitor, or pharmaceutically acceptable salts thereof.

In still another aspect, the present invention provides a use of (i) an angiotensin receptor blocker, or pharmaceutically acceptable salts thereof; and (ii) an SGLT2 inhibitor, or pharmaceutically acceptable salts thereof in the manufacture of a medicament for preventing or treating hypertension. The angiotensin receptor blocker, SGLT2 inhibitor, salts, and the like, are as described above.

Matters described in all compositions, combinations, treatment methods, and uses of the present invention are equally applied unless they contradict each other.

Hereinafter, the constitution and effects of the present invention will be described in more detail through the following Examples. These Examples are only provided for illustrating the present invention, but the scope of the present invention is not limited by these Examples.

### <Example 1> Preparation of animal model of hypertension

### Reagent

Drugs administered in experimental animals were azilsartan medoxomil, fimasartan potassium trihydrate, candesartan cilexetil, olmesartan medoxomil, or telmisartan as angiotensin receptor blockers and dapagliflozin propanediol hydrate or empagliflozin as SGLT2 inhibitors.

### Experimental animals

The spontaneously hypertensive rat (SHR), a disease animal model that is well correlated with human hypertension patients, was used. Specifically, 5.5-week-old male SHRs were purchased from Charles River and used in the experiments after an acclimatization period. The animals were housed in a room with a temperature of 22 ± 5°C, relative humidity of 50 ± 10%, and a 12-hour light/dark cycle, and food and water were provided ad libitum.

### Data analysis

All data are expressed as mean ± S.E.M. Blood pressure measurements were schematized using GraphPad Prism 9 (GraphPad Software Inc., San Diego, CA).

### <Example 2> Exploring combination dose of angiotensin receptor blocker and SGLT2 inhibitor

The experiments were conducted using 15-28 week-old SHR (300-380 g), and SHR with systolic blood pressure of 190 mmHg or more were used in the experiment as a result of measuring basic blood pressure.

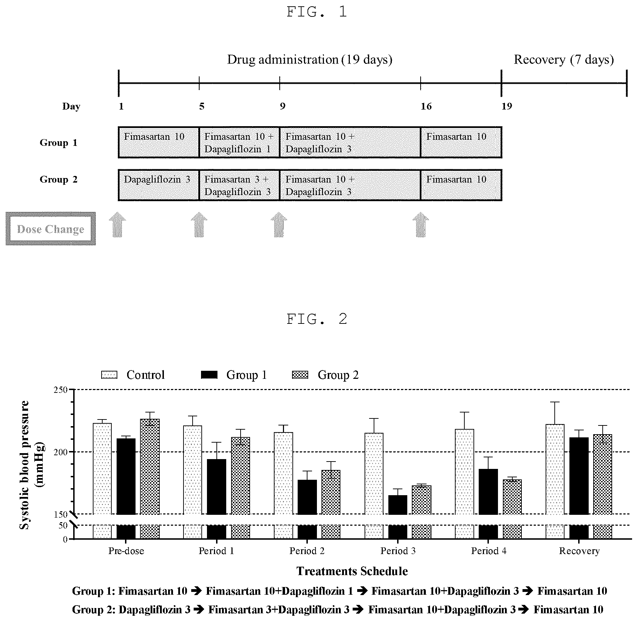

The animals were divided into two groups (4 animals per group) and administered with fimasartan and dapagliflozin at single or combined doses (mg/kg/day), while observing the changes in blood pressure, with dose changes according to the schedule shown in FIG. 1. In addition, the drug was administered orally at a volume of ul per g body weight at the same time once daily.

Blood pressure was measured daily immediately before (0 hr) and 4 hours after (4 hr) dosing while administering the drug for a total of 19 days, and the recovery of blood pressure was monitored during a 1-week recovery period after the end of administration. Specifically, the animals were divided into two groups to evaluate the blood pressure response when fimasartan (Group 1) or dapagliflozin (Group 2) was administered alone, and to evaluate the blood pressure response to the addition of the other drugs in sequentially increasing doses, and the results are shown in FIG. 2.

As shown in FIG. 2, the administration of dapagliflozin alone had a modest blood pressure-lowering effect, but when combined with fimasartan, the blood pressure-lowering effects of each drug were found to be enhanced. In particular, the combined administration of fimasartan 3 mg/kg/day + dapagliflozin 3 mg/kg/day had a blood pressure-lowering effect similar to that of the maximum dose of fimasartan, 10 mg/kg/day.

### <Example 3> Confirmation of blood pressure-lowering effect of combined administration of angiotensin receptor blocker and dapagliflozin

The results of Example 2 above confirmed that dapagliflozin does not have a significant blood pressure-lowering effect on its own, but enhances the blood pressure-lowering action of fimasartan when combined administered with a therapeutic agent for treating hypertension such as fimasartan. Thus, the effects of dapagliflozin on blood pressure when administered in combination with various angiotensin receptor blockers were compared and evaluated.

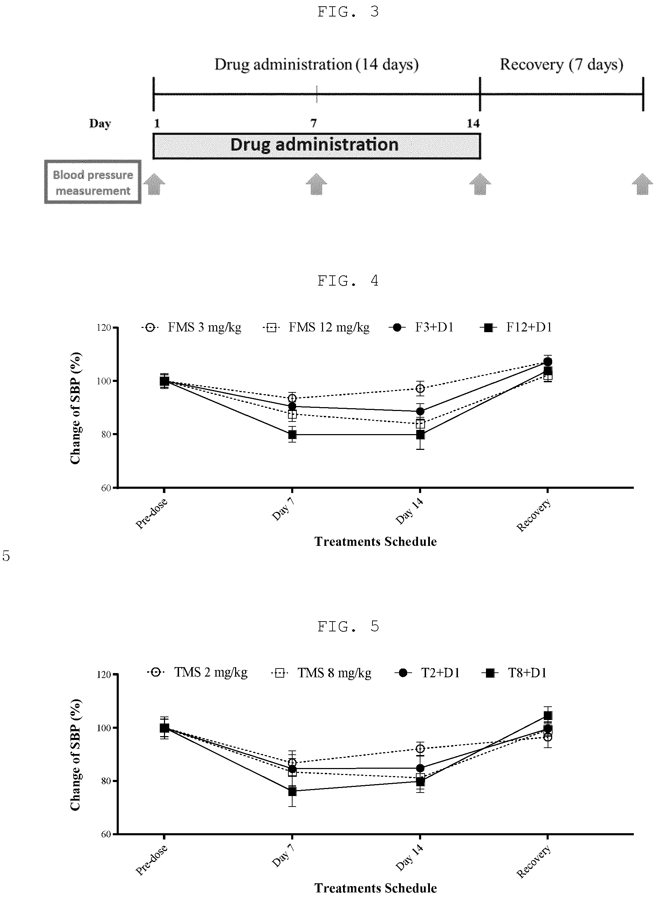

Specifically, the experiments were conducted for a total of 14 days using the same method as in Example 2 above, with the schedule shown in FIG. 3, and each experimental group was organized as shown in Table 1 below, with 8-10 animals per group.

**[Table 1]**

| **Experimental Group** | **Reagent** |
|---|---|
| Solvent control group | D.W. |
| F3 | Fimasartan 3 mg/kg/day |
| F12 | Fimasartan 12 mg/kg/day |
| F3+D1 | Fimasartan 3 mg/kg/day + Dapagliflozin 1 mg/kg/day |
| F12+D1 | |
| | Fimasartan 12 mg/kg/day + Dapagliflozin 1 mg/kg/day |
| | |
| T2 | Telmisartan 2 mg/kg/day |
| T8 | Telmisartan 8 mg/kg/day |
| T2+D1 | Telmisartan 2 mg/kg/day + Dapagliflozin 1 mg/kg/day |
| T8+D1 | |
| | Telmisartan 8 mg/kg/day + Dapagliflozin 1 mg/kg/day |
| | |
| C0.8 | Candesartan 0.8 mg/kg/day |
| C3.2 | Candesartan 3.2 mg/kg/day |
| C0.8+D1 | Candesartan 0.8 mg/kg/day + Dapagliflozin 1 mg/kg/day |
| C3.2+D1 | |
| | Candesartan 3.2 mg/kg/day + Dapagliflozin 1 mg/kg/day |
| | |

The angiotensin receptor blockers, fimasartan, telmisartan, and candesartan were divided into low and high doses (4 times the low dose), and each administered alone or in combination with dapagliflozin, and the results of blood pressure measurements in each experimental group are shown in Table 2 and FIGS. 4 to 6 below.

**[Table 2]**

| | | Day 1 (pre-dose) | Day 7 (4h) | Day 14 (4h) | Recovery |
|---|---|---|---|---|---|
| Solvent control group (D.W.) | Mean | 100.0 | 99.9 | 106. | 102.6 |
| | S.E.M. | 2.4 | 3.2 | 2.5 | 0.9 |
| Dapagliflozin 1 mg/kg/day | Mean | 100.0 | 101.2 | 96.5 | 105.5 |
| | S.E.M. | 2.3 | 2.2 | 3.3 | 1.2 |
| Fimasartan 3 mg/kg/day | Mean | 100.0 | 93.5 | 97.2 | 107.2 |
| | S.E.M. | 2.4 | 2.2 | 2.6 | 2.2 |
| Fimasartan 12 mg/kg/day | Mean | 99.4 | 90.4 | 86.2 | 102.2 |
| | S.E.M. | 1.1 | 3.2 | 2.6 | 2.0 |
| Fimasartan 3 mg/kg/day + Dapagliflozin 1 mg/kg/day | Mean | 100.0 | 93.9 | 89.8 | 107.2 |
| | S.E.M. | 2.8 | 3.2 | 2.5 | 2.2 |
| Fimasartan 12 mg/kg/day + Dapagliflozin 1 mg/kg/day | Mean | 100.4 | 81.6 | 80.0 | 104.0 |
| | S.E.M. | 2.3 | 3.7 | 5.5 | 4.1 |
| Telmisartan 2 mg/kg/day | Mean | 100.0 | 86.8 | 92.2 | 96.6 |
| | S.E.M. | 0.9 | 3.1 | 2.2 | 3.5 |
| Telmisartan 8 mg/kg/day | Mean | 100.0 | 83.4 | 81.3 | 99.4 |
| | S.E.M. | 3.2 | 5.1 | 3.9 | 2.5 |
| Telmisartan 2 mg/kg/day + Dapagliflozin 1 mg/kg/day | Mean | 100.0 | 84.6 | 84.9 | 99.7 |
| | S.E.M. | 3.4 | 5.8 | 4.2 | 2.4 |
| Telmisartan 8 mg/kg/day + Dapagliflozin 1 mg/kg/day | Mean | 100.0 | 76.2 | 79.9 | 104.7 |
| | S.E.M. | 3.6 | 5.0 | 3.9 | 3.0 |
| Candesartan 0.8 mg/kg/day | Mean | 100.0 | 90.5 | 98.0 | 104.5 |
| | S.E.M. | 2.6 | 2.00 | 4.0 | 2.9 |
| Candesartan 3.2 mg/kg/day | Mean | 100.0 | 86.6 | 86.9 | 104.4 |
| | S.E.M. | 3.7 | 4.6 | 3.8 | 1.2 |
| Candesartan 0.8 mg/kg/day + Dapagliflozin 1 mg/kg/day | Mean | 100.0 | 82.7 | 84.8 | 98.1 |
| | S.E.M. | 2.6 | 3.0 | 5.1 | 1.5 |
| Candesartan 3.2 mg/kg/day + Dapagliflozin 1 mg/kg/day | Mean | 100.0 | 79.1 | 84.2 | 106.1 |
| | S.E.M. | 2.6 | 3.2 | 3.3 | 2.0 |

As shown in Table 2 and FIGS. 4 to 6, the blood pressure-lowering effect of all of the above ARBs was enhanced when administered in combination with dapagliflozin compared to the administration of low or high doses alone, and in particular, the administration of a low dose of the ARB in combination with dapagliflozin exhibited a blood pressure-lowering effect similar to that of a high dose of an ARB alone.

The above results demonstrate that the combined administration of an angiotensin receptor blocker and an SGLT2 inhibitor, dapagliflozin, may have a synergistic effect on blood pressure lowering, and reduce the dosage of an angiotensin receptor blocker which is a therapeutic agent for treating hypertension, indicating that the composite composition of the present invention may be useful for the therapeutic agent for treating hypertension.

### <Example 4> Confirmation of blood pressure-lowering effect following combined administration of angiotensin receptor blocker and empagliflozin

The experiments were conducted in the same manner as in Example 3 above, but using empagliflozin as an SGLT2 inhibitor. Each experimental group was organized as shown in Table 3 below, with 8-10 animals per group.

**[Table 3]**

| **Experimental Group** | **Reagent** |
|---|---|
| Solvent control group | D.W. |

| E1 | Empagliflozin 1 mg/kg/day |
|---|---|
| A2 | Azilsartan 2 mg/kg/day |
| A8 | Azilsartan 8 mg/kg/day |
| A2+E1 | Azilsartan 2 mg/kg/day + Empagliflozin 1 mg/kg/day |
| A8+E1 | |
| | Azilsartan 8 mg/kg/day + Empagliflozin 1 mg/kg/day |
| O1 | Olmesartan 1 mg/kg/day |
| O4 | Olmesartan 4 mg/kg/day |
| O1+E1 | Olmesartan 1 mg/kg/day + Empagliflozin 1 mg/kg/day |
| O4+E1 | |
| | Olmesartan 4 mg/kg/day + Empagliflozin 1 mg/kg/day |
| | |
| T1 | Telmisartan 1 mg/kg/day |
| T2 | Telmisartan 2 mg/kg/day |
| T8 | Telmisartan 8 mg/kg/day |
| T1+E1 | Telmisartan 1 mg/kg/day + Empagliflozin 1 mg/kg/day |
| T2+E1 | |
| T8+E1 | Telmisartan 2 mg/kg/day + Empagliflozin 1 mg/kg/day |
| | Telmisartan 8 mg/kg/day + Empagliflozin 1 mg/kg/day |

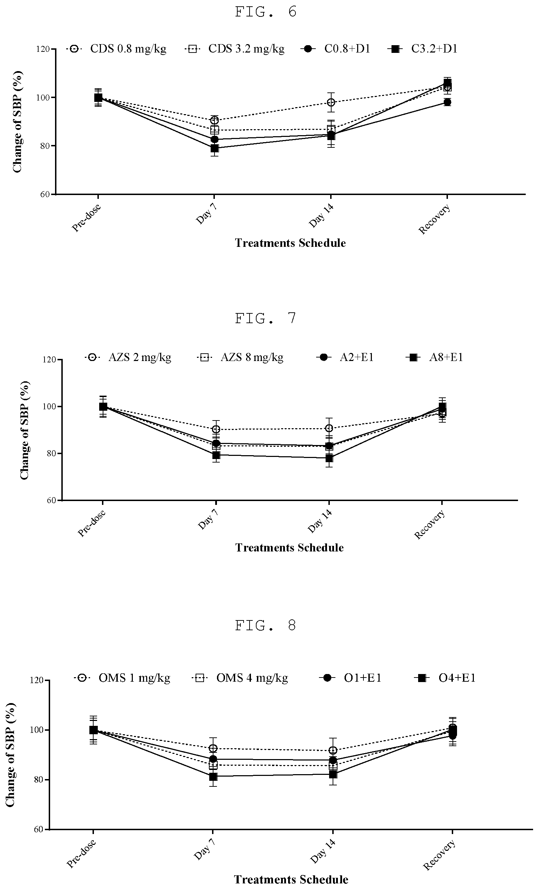

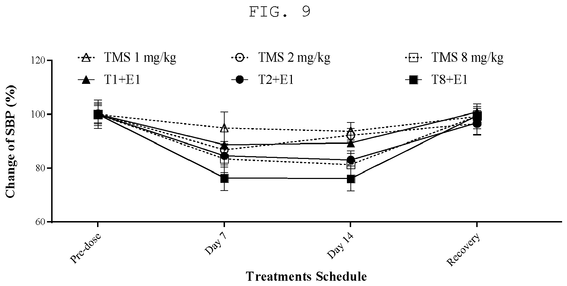

The angiotensin receptor blockers, fimasartan, azilsartan, and olmesartan were divided by dose, and each administered alone or in combination with empagliflozin, and the results of blood pressure measurements in each experimental group are shown in Table 4 and FIGS. 7 to 9 below.

**[Table 4]**

| | | Day 1 (pre-dose) | Day 7 (4h) | Day 14 (4h) | Recovery |
|---|---|---|---|---|---|
| Solvent control group (D.W.) | Mean | 100.0 | 99.9 | 106.0 | 102.6 |
| | S.E.M. | 2.4 | 3.2 | 2.5 | 0.9 |
| Empagliflozin 1 mg/kg/day | Mean | 100.0 | 97.1 | 96.4 | 101.8 |
| | S.E.M. | 4.2 | 3.4 | 5.2 | 3.6 |
| Azilsartan 2 mg/kg/day | Mean | 100.0 | 94.9 | 93.7 | 99.2 |
| | S.E.M. | 3.6 | 5.9 | 3.3 | 4.6 |
| Azilsartan | Mean | 100.0 | 86.8 | 92.2 | 96.6 |

| 8mg/kg/day | S.E.M. | 0.8 | 2.7 | 2.0 | 3.2 |
|---|---|---|---|---|---|
| Azilsartan 2 mg/kg/day + Empagliflozin 1 mg/kg/day | Mean | 100.0 | 83.4 | 81.3 | 99.4 |
| | S.E.M. | 2.8 | 4.6 | 3.5 | 2.2 |
| Azilsartan 8 mg/kg/day + Empagliflozin 1 mg/kg/day | Mean | 100.0 | 88.6 | 89.3 | 100.8 |
| | S.E.M. | 3.9 | 5.2 | 4.1 | 4.0 |
| Olmesartan 1 mg/kg/day | Mean | 100.0 | 84.6 | 83.0 | 96.8 |
| | S.E.M. | 5.3 | 4.3 | 3.3 | 4.5 |
| Olmesartan 4 mg/kg/day | Mean | 100.0 | 76.4 | 76.1 | 99.4 |
| | S.E.M. | 4.2 | 4.7 | 4.6 | 3.4 |
| Olmesartan 1 mg/kg/day + Empagliflozin 1 mg/kg/day | Mean | 100.0 | 92.6 | 91.8 | 100.9 |
| | S.E.M. | 5.6 | 4.2 | 5.0 | 2.5 |
| Olmesartan 4 mg/kg/day + Empagliflozin 1 mg/kg/day | Mean | 100.0 | 86.0 | 85.7 | 99.5 |
| | S.E.M. | 3.8 | 5.0 | 3.6 | 5.2 |
| Telmisartan 1 mg/kg/day | Mean | 100.0 | 88.3 | 87.8 | 97.7 |
| | S.E.M. | 4.8 | 4.3 | 3.0 | 4.0 |
| Telmisartan 2 mg/kg/day | Mean | 100.0 | 81.3 | 82.3 | 100.2 |
| | S.E.M. | 3.9 | 4.0 | 4.4 | 4.8 |
| Telmisartan 8 mg/kg/day | Mean | 100.0 | 90.3 | 90.7 | 97.0 |
| | S.E.M. | 4.2 | 3.8 | 4.3 | 3.8 |
| Telmisartan 1 mg/kg/day + Empagliflozin 1 mg/kg/day | Mean | 100.0 | 83.4 | 83.1 | 97.6 |
| | S.E.M. | 3.2 | 3.7 | 4.5% | 3.1 |
| Telmisartan 2 mg/kg/day + Empagliflozin 1 mg/kg/day | Mean | 100.0 | 84.4 | 83.3 | 98.9 |
| | S.E.M. | 4.2 | 3.9 | 3.5 | 3.7 |
| Telmisartan 8 mg/kg/day + Empagliflozin 1 mg/kg/day | Mean | 100.0 | 79.4 | 78.1 | 100.2 |
| | S.E.M. | 4.6 | 3.0 | 3.8 | 3.7 |

As shown in Table 4 and FIGS. 7 to 9, the blood pressure-lowering effect of all of the above ARBs was enhanced when administered in combination with empagliflozin compared to the administration of all doses of the ARBs alone, and in particular, the administration of a low dose of the ARB in combination with empagliflozin exhibited a blood pressure-lowering effect similar to that of a high dose of an ARB alone.

The above results demonstrate that the combined administration (co-administration) of an angiotensin receptor blocker and an SGLT2 inhibitor, empagliflozin, may have a synergistic effect on blood pressure lowering, and reduce the dosage of an angiotensin receptor blocker which is a therapeutic agent for treating hypertension, indicating that the composite composition of the present invention may be useful for the therapeutic agent for treating hypertension.

## Claims

1. A pharmaceutical composition for preventing or treating hypertension, comprising:
(i) an angiotensin receptor blocker selected from the group consisting of fimasartan, telmisartan, candesartan, azilsartan, olmesartan, and pharmaceutically acceptable salts thereof; and
(ii) a sodium-glucose co-transporter subtype 2 (SGLT2) inhibitor selected from the group consisting of dapagliflozin, empagliflozin, and pharmaceutically acceptable salts thereof.

2. The pharmaceutical composition of claim 1, wherein a weight ratio of the angiotensin receptor blocker and the SGLT2 inhibitor is 0.5 : 1 to 15 : 1.

3. The pharmaceutical composition of claim 1, wherein the composition is orally administered.

4. A combination for preventing or treating hypertension comprising:
(i) a first agent comprising an angiotensin receptor blocker selected from the group consisting of fimasartan, telmisartan, candesartan, azilsartan, olmesartan, and pharmaceutically acceptable salts thereof; and
(ii) a second agent comprising a sodium-glucose co-transporter subtype 2 (SGLT2) inhibitor selected from the group consisting of dapagliflozin, empagliflozin, and pharmaceutically acceptable salts thereof.

5. The combination of claim 4, wherein the first agent and the second agent are administered either simultaneously or at different times.

6. The combination of claim 4, wherein the combination is a composite preparation comprising the first agent and the second agent.

7. A pharmaceutical adjuvant composition for preventing, treating, or improving hypertension, or lowering blood pressure of an angiotensin receptor blocker, comprising: a sodium-glucose co-transporter subtype 2 (SGLT2) inhibitor or pharmaceutically acceptable salts thereof.

8. The pharmaceutical adjuvant composition of claim 7, wherein the SGLT2 inhibitor is selected from the group consisting of dapagliflozin, empagliflozin, and pharmaceutically acceptable salts thereof.

9. The pharmaceutical adjuvant composition of claim 7, wherein the angiotensin receptor blocker is selected from the group consisting of fimasartan, telmisartan, candesartan, azilsartan, olmesartan, and pharmaceutically acceptable salts thereof.

10. A food composition for improving hypertension or lowering blood pressure, comprising:
(i) an angiotensin receptor blocker selected from the group consisting of fimasartan, telmisartan, candesartan, azilsartan, olmesartan, and pharmaceutically acceptable salts thereof; and
(ii) a sodium-glucose co-transporter subtype 2 (SGLT2) inhibitor selected from the group consisting of dapagliflozin, empagliflozin, and pharmaceutically acceptable salts thereof.
